Europäisches Patentamt

European Patent Office

Office européen des brevets

(19)

(11) Veröffentlichungsnummer: **0 184 755**
**A2**

(12) # EUROPÄISCHE PATENTANMELDUNG

(21) Anmeldenummer: 85115285.0

(22) Anmeldetag: 02.12.85

(51) Int. Cl.⁴: **C 12 P 19/04**
// D06P1/48, C09D7/00,
C12R1:22

(30) Priorität: 12.12.84 DE 3445302

(43) Veröffentlichungstag der Anmeldung: 18.06.86
Patentblatt 86/25

(84) Benannte Vertragsstaaten: AT BE CH DE FR GB IT LI NL
SE

(71) Anmelder: HOECHST AKTIENGESELLSCHAFT,
Postfach 80 03 20, D-6230 Frankfurt am Main 80 (DE)

(72) Erfinder: Voelskow, Hartmut, Dr., Akazienstrasse 22,
D-6234 Hattersheim am Main (DE)
Erfinder: Schlingmann, Merten, Dr., Schneidhainer
Strasse 32a, D-6240 Königstein/Taunus (DE)

(54) Extrazelluläres Polysaccharid, Verfahren und Stamm zur seiner Herstellung und seine Verwendung.

(57) Durch Fermentation des Bakterienstammes Klebsiella planticola DSM 3092 erhält man ein extrazelluläres Polysaccharid, das als Viskositätsregulator in wäßrigen Systemen, beispielsweise in Gips- und Putzmassen, dienen kann. Es eignet sich auch als Verdickungsmittel, wenn hohe Viskositäten gefordert werden, beispielsweise in Textildruckpasten.

EP 0 184 755 A2

Extrazelluläres Polysaccharid, Verfahren und Stamm zu
seiner Herstellung und seine Verwendung

Die Erfindung betrifft ein neues, extrazelluläres Polysaccharid, das von einem Bakterienstamm der Art Klebsiella
planticola gebildet wird, ein Fermentationsverfahren zur
Herstellung dieses Exopolysaccharids, den Bakterienstamm,
der unter der Nummer DSM 3092 hinterlegt ist und die Verwendung des Polysaccharids als Viskositätsregulator für
wäßrige Systeme.

Es sind bereits extrazelluläre Polysaccharide beschrieben,
die von Bakterien der Gattung Klebsiella erzeugt werden,
und zwar von Stämmen der Art Klebsiella pneumoniae: Aus der
US-Patentschrift 4 298 691 ist ein Exopolysaccharid bekannt, das Fucose und Galacturonsäure enthält. Aus der US-
Patentschrift 4 350 769 ist ein ähnliches Produkt bekannt,
dessen Kohlenhydratfraktion zu 51 % aus Glucose und zu
49 % aus Fucose besteht.

Gegenüber diesen bekannten Exopolysacchariden unterscheidet sich das erfindungsgemäße extrazelluläre Polysaccharid
durch einen Gehalt an Mannose anstelle von Fucose und durch
einen geringen Glucosegehalt. Diese Unterschiede sind
überraschend, da die beiden Klebsiella-Arten sehr nahe
verwandt sind.

Der Stamm DSM 3092 wurde aus einer Erdprobe isoliert, die
bei Niedernhausen/Taunus gefunden wurde. Er kann bei 30°C
in einem üblichen Glucose-Pepton-Medium aerob kultiviert
werden.

In den Produktionsmedien kann Saccharose oder Lactose als
Kohlenstoffquelle dienen. Die Temperatur liegt zweckmäßig im Bereich von 25 bis 35°C, insbesondere bei etwa 30°C, der pH-Wert im schwach sauren bis neutralen Bereich, zweckmäßig bei pH 6 bis 7,5, insbesondere bei 6,8. Nach etwa 3 Tagen werden pro Liter Kulturmedium 20 bis 25 g Exopolysaccharid gebildet.

Die Isolierung des Exopolysaccharids erfolgt in an sich bekannter Weise, beispielsweise durch Fällen mit polaren wassermischbaren organischen Lösemitteln wie Alkanolen oder Alkanonen, vor allem mit Aceton oder Alkanolen mit 1 bis 3 Kohlenstoffatomen. Für technische Zwecke kann auch eine Fällung als Addukt mit einer langkettigen Aminverbindung erfolgen. Hierfür eignen sich primäre, sekundäre und tertiäre Amine mit einem langkettigen Kohlenwasserstoffrest, vorteilhaft handelsübliche Fettamine, mit einem Kohlenwasserstoffrest von 12 bis 30, insbesondere 16 bis 24 Kohlenstoffatomen. Im Falle der sekundären und tertiären Amine tragen diese einen oder zwei kurzkettige Alkylreste, insbesondere Methylgruppen. Geeignet sind auch quarternäre Ammoniumsalze mit einem der genannten langkettigen Kohlenwasserstoffreste und drei der angeführten kurzkettigen Alkylgruppen sowie Derivate von Polyaminen vom Typ der Oligo-ethylendiamine. Aus diesen Aminaddukten können gewünschtenfalls die freien Polysaccharide in bekannter Weise mit Basen freigesetzt werden.

Gegenüber dem bekanntesten handelsüblichen Exopolysaccharid, Xanthan Gum, zeichnet sich das erfindungsgemäße Polymer dadurch aus, daß seine Lösungen in Wasser oder Salzlösungen bei höheren Konzentrationen deutlich höhere Viskositäten zeigen. Hierdurch ist das erfindungsgemäße Exopolysaccharid dem Xanthan ganz allgemein als Verdickungsmittel überlegen, wenn eine hohe Viskosität gefordert wird. Besondere Vorteile zeigen sich bei der Korrektur des Fließverhaltens von Gips- und Putzmischungen. Das höhere Wasserretentionsvermögen wird beim Einarbeiten in Textildruckfarben deutlich, wo das kapillare Verlaufen verhindert wird.

In den folgenden Beispielen wird die Erfindung näher erläutert. Prozentangaben beziehen sich auf das Gewicht, wenn keine anderen Angaben gemacht sind.

<u>Beispiel 1</u>

Von einer Schrägagarkultur des Stammes DSM 3092 werden mit einer Impföse 100 ml Glucose-Pepton-Medium der Zusammensetzung pro Liter

5 g Glucose,

2 g Caseinpepton,

2 g Fleischextrakt,

0,25 g Hefeextrakt,

0,25 g Leberextrakt und

1,25 g NaCl

beimpft und bei 30°C aerob bebrütet. Nach 48 Stunden werden diese 100 ml Kulturlösung in einen Fermenter überführt, in dem 16 l Produktionsmedium der folgenden Zusammensetzung (pro Liter) vorgelegt sind:

45 g Saccharose,

2,5 g Cornsteep, trocken,

2 g $NaNO_3$,

2 g $K_2HPO_4$ und

1,5 g $MgSO_4$.

Der Fermenter wird mit einem Volumen Luft pro Volumen Kulturmedium und Minute belüftet und mit einem Wendelrührer mit 500 UpM gerührt. Die Temperatur wird auf 30°C eingestellt und der pH-Wert durch Natronlauge-Zugabe auf 6,8 gehalten. Nach 72 Stunden werden in der Kultur 21 g Polysaccharid pro Liter Medium gebildet.

Der Fermenterinhalt wird 2 Minuten auf 95°C erhitzt, auf etwa 50°C abgekühlt und dann mit dem 1,2fachen Volumen Isopropanol versetzt. Das dabei ausgefallene Polysaccharid wird abfiltriert und getrocknet.

Eine Probe des Polysaccharids wird 5 Stunden bei 100°C mit 2 N Trifluoressigsäure hydrolysiert. Durch Hochdruckflüssigchromatographie (HPLC, Säule HPX-87 C) wird folgende Zusammensetzung ermittelt:

| | |
|---|---|
| Mannose | 15,9 % |
| Galactose | 38,2 % |
| Glucose | 1,7 % |
| Glucuronsäure-lacton | 17,5 % |
| Essigsäure | 5,3 % |
| Bernsteinsäure | 2,6 % |
| Sulfatasche | 14,6 % |

Die Viskosität (in mPa.s) im Vergleich zu Xanthan Gum (Handelsprodukt [R]RHODOPOL 23 der Firma Rhone Poulenc) wurde wie folgt bestimmt:

| Scher-gefälle ($sec^{-1}$) | Produkt | Konzentration (%) in | | | | | |
|---|---|---|---|---|---|---|---|
| | | Wasser | | | Salzlösung (1 % $CaCl_2$, 13 % NaCl) | | |
| | | 0,2 | 0,5 | 1 | 0,2 | 0,5 | 1 |
| 424 | Erfindung | 12 | 42 | 142 | 12 | 31 | 92 |
| | Xanthan | 10 | 29 | 67 | 12 | 26 | 52 |
| 10 | Erfindung | 31 | 243 | 2228 | 23 | 210 | 2529 |
| | Xanthan | 87 | 404 | 1475 | 88 | 381 | 1305 |

**Beispiel 2**

Eine Kultur des Stammes DSM 3092 wird - wie im Beispiel 1 beschrieben - in das Glucose-Pepton-Medium verimpft und bebrütet. Bei der anschließenden Fermentation wird jedoch im Produktionsmedium Lactose anstelle der Saccharose eingesetzt und 90 Stunden bebrütet. Ausfällung und Trocknung erfolgen wieder nach Beispiel 1. Man erhält ein Exopolysaccharid, dessen rheologische Daten nur minimal von den im Beispiel 1 genannten abweichen.

**Anhang:** Identifizierung des Stammes DSM 3092 durch die Deutsche Sammlung von Mikroorganismen:

| Zellform | Stäbchen |
|---|---|
| Länge µm | 1,2-2,0 |
| Breite µm | 0,8-1,0 |
| Beweglichkeit | – |
| Geißeln | – |
| Gram-Reaktion | – |
| Sporen | – |
| Oxidase | – |
| Catalase | + |
| Wachstum | |
| anaerob | + |
| 44,5°C | + |
| 10°C | + |
| 4°C | – |
| KCN | + |
| Mac-Conkey-Agar | + |
| SS-Agar | + |
| Cetrimid-Agar | + |

0184755

Pigmentbildung

  nicht-

  diffundierend       -

  diffundierend         -

  fluoreszierend       -


Säurebildung

(OF-Test) aus

  Glucose aerob       +

          anaerob   +


Gasbildung aus

Glucose              +


FC-Test             -

(Gasbildung aus Lactose in

EC-Bouillon bei 44,5°C)

Säure aus

  Adonit            -

  L-Arabinose       +

  Cellobiose       +

  Dulcit            +

  Glycerin         +

  Inosit            +

  Lactose          +

  Maltose          +

  Mannit            +

  L-Sorbose        +

  D-Melezitose      -

  Melibiose        +

  Raffinose        +

  Rhamnose         +

  Salicin          +

  Sorbit           +

  Saccharose       +

  Trehalose        +

  D-Xylose         +

  Inulin            -

0184755

| | |
|---|---|
| ONPG | + |
| Arginindihydrolase | - |
| Lysindecarboxylase | + |
| Ornithindecarboxylase | - |
| $H_2S$ (TSI) | - |
| Voges-Proskauer | + |
| Methylrot | - |
| Indol | - |
| Nitrit aus Nitrat | + |
| Phenylalanindeaminase | - |
| Urease | + |
| Abbau von | |
| Gelatine | - |
| DNA | - |
| Äsculin | + |
| Pectin | - |
| | |
| Substratverwertung | |
| Acetat | + |
| Citrat | + |
| Malonat | + |
| Arginin | + |
| Hydroxyprolin | + |
| $\gamma$-Aminobutyrat | + |
| L-Phenylalanin | + |
| | |
| Jordans's Tartrat | + |

PATENTANSPRÜCHE:

1. Extrazelluläres Polysaccharid, erhältlich durch Fermentation des Stammes Klebsiella planticola DSM 3092.

2. Extrazelluläres Polysaccharid, gekennzeichnet durch einen Gehalt von

| | |
|---|---|
| 15,9 % | Mannose |
| 38,2 % | Galactose |
| 1,7 % | Glucose |
| 17,5 % | Glucuronsäure-lacton |
| 5,3 % | Essigsäure |
| 2,6 % | Bernsteinsäure |
| 14,6 % | Sulfatasche. |

3. Verfahren zur Herstellung eines extrazellulären Polysaccharids durch Fermentation von Bakterien der Gattung Klebsiella, dadurch gekennzeichnet, daß man den Stamm Klebsiella planticola DSM 3092 kultiviert.

4. Klebsiella planticola DSM 3092 .

5. Verwendung des extrazellulären Polysaccharids nach Anspruch 1 oder 2 bzw. des nach Anspruch 3 erhältlichen Polysaccharids als Viskositätsregulator in wäßrigen Systemen.

6. Verwendung des extrazellulären Polysaccharids nach Anspruch 1 oder 2 bzw. des nach Anspruch 3 erhältlichen Polysaccharids als Verdickungsmittel in Textildruckpasten.

HOE 01845755

**Patentansprüche:** Österreich

1. Verfahren zur Herstellung eines extrazellulären Polysaccharids durch Fermentation von Bakterien der Gattung Klebsiella, dadurch gekennzeichnet, daß der Stamm Klebsiella planticola DSM 3092 kultiviert wird bis sich ein Polysaccharid anhäuft

   mit einem Gehalt von
   15,9% Mannose
   38,2% Golaetose
    1,7% Glucose
   17,5% Glucuronsäurelacton
    5,3% Essigsäure
    2,6% Bernsteinsäure
   14,6% Sulfatasche

   und das genannte Polysaccharid gegebenenfalls isoliert wird.

2. Klebsiella planticola DSM 3092.

3. Verwendung des extrazellulären Polysaccharids, erhältlich nach Anspruch 1, als Viskositätsregulator in wäßrigen Systemen.

4. Verwendung des extrazellulären Polysaccharids, erhältlich nach Anspruch 1, als Verdickungsmittel in Textildruckpasten.

**Europäisches Patentamt**

0184755
Anmeldenummer:

*85115285.0*

# ERKLÄRUNG NACH REGEL 28 ABSATZ 4 DES EUROPÄISCHEN PATENTÜBEREINKOMMENS

Der Anmelder hat dem Europäischen Patentamt mitgeteilt, daß der in Regel 28 Absatz 3 des Europäischen Patentübereinkommens vorgesehene Zugang zu den nachstehend gekennzeichneten Mikroorganismen bis zu dem Tag, an dem der Hinweis auf die Erteilung des europäischen Patents bekanntgemacht wird oder an dem die Patentanmeldung zurückgewiesen oder zurückgenommen wird oder als zurückgenommen gilt, nur durch die Herausgabe einer Probe an einen Sachverständigen hergestellt wird.

## KENNZEICHNUNG DER MIKROORGANISMEN

**Eingangsnummern der Hinterlegungen:**  *DSM 3092*